# EUROPEAN PATENT SPECIFICATION

(11) **EP 4 242 650 B1**
(45) Date of publication and mention of the grant of the patent: **03.09.2025**
(21) Application number: 23161049.4
(22) Date of filing: 09.03.2023
(51) Int. Cl.: G01N 33/00, G01N 27/407

(54) **CARBON MONOXIDE TRANSDUCER**
KOHLENMONOXIDWANDLER
TRANSDUCTEUR DE MONOXYDE DE CARBONE

(30) Priority: 10.03.2022 CN 202220512943 U
(43) Date of publication of application: 13.09.2023
(73) Proprietor: Carrier Corporation, Palm Beach Gardens, FL 33418 (US)
(72) Inventor: SUN, Pengfei, Guangdong, 523800 (CN)
(74) Representative: Dehns

(56) References cited:
- JP-A- 2002 350 393
- JP-A- 2011 220 716
- US-A- 4 406 770
- US-A1- 2003 145 644

## Description

The present invention relates to the field of gas transducers, in particular to a carbon monoxide transducer.

Carbon monoxide transducers are commonly used in various fields for detection of carbon monoxide leakage, the concept of which is to monitor the voltage generated by the reaction of carbon monoxide and water. The existing carbon monoxide transducers generally include a water chamber to contain water for reaction with carbon monoxide. In order to meet the designed life of the carbon monoxide transducer (which can be as long as ten years), the water chamber generally includes a relatively large volume, which leads to a relatively large volume of the entire carbon monoxide transducer.

The object of the present application is to solve or at least alleviate the problems existing in the prior art.

JP 2002 350 393 A discloses a sensor main body employing a proton conductive film arranged between a metallic washer and a covering body and held by a metallic can through a dielectric gasket.

US 4 406 770 A discloses a gas sensor cell assembly having planar electrodes separated by a planar hydrophilic wick which extends through the electrode into an electrolyte expansion chamber where it contacts an electrolyte.

US 2003/0145644 A1 discloses a self-calibrating carbon monoxide detector that utilizes the gas for which the detector was designed to detect as the calibration gas.

JP 2011 220716 A discloses a filter used for a gas sensor.

According to the invention, a carbon monoxide transducer is provided, which comprises:
a main housing;
a lower support plate arranged in the main housing, wherein the lower support plate divides the main housing into a water chamber containing water below the lower support plate and a reaction chamber above the lower support plate;
a multilayer film assembly arranged in the reaction chamber on the lower support plate; and
a cover assembly on the multilayer film assembly, wherein the cover assembly comprises a top cover, an upper support plate, a filter body between the top cover and the upper support plate, and a sealing ring wrapped around an outer periphery of the top cover and the upper support plate;
wherein, a curling portion is formed at the top of the main housing that compresses the sealing ring of the cover assembly and the multilayer film assembly towards the lower support plate,
wherein the multilayer film assembly comprises a first insulating diffusion layer on the lower support plate and a reaction film on the first insulating diffusion layer, a ring washer on the reaction film, and a second insulating diffusion layer, wherein the second insulating diffusion layer is located in a center hole of the ring washer, and an outer diameter of the ring washer is close to an inner diameter of the reaction chamber, and
wherein the upper support plate abuts against the second insulating diffusion layer, the bottom of the sealing ring abuts against the ring washer, and an outer edge of the ring washer is pressed down to abut against the lower support plate.

Optionally, the sealing ring is made of natural rubber, and the curling portion is arranged to compress the sealing ring and the multilayer film assembly, so that the total thickness of the sealing ring and the multilayer film assembly is compressed by 20% to 40%.

Optionally, the curling portion is arranged to compress the sealing ring and the multilayer film assembly, so that the total thickness of the sealing ring and the multilayer film assembly is compressed by 25% to 35%.

Optionally, the total thickness of the multilayer film assembly is compressed by at least 40%.

Optionally, the inner side of the main housing has a shoulder, the lower support plate is arranged on the shoulder of the main housing, and the lower support plate and the main housing are made of conductive materials and are connected to each other.

Optionally, the top cover comprises a middle protruding portion and a plane portion at the outside of the middle protruding portion, the upper support plate is formed into a flat plate, the plane portion of the top cover abuts against the upper support plate, and the outer periphery is wrapped by the sealing ring. The top cover and the upper support plate are made of conductive materials and are connected to each other. The filter body is located in a chamber defined by the protruding portion of the top cover, the filter body is an activated carbon bag, and a gasket is further arranged between the filter body and the upper support plate.

Optionally, electrode sheets that are attached to the top cover and the bottom of the main housing respectively are also included.

Optionally, the diameter of the water chamber is less than 15mm and the height of the water chamber is less than 30mm.

The carbon monoxide transducer according to the present invention has stronger sealing performance, which can reduce the volume of the water chamber and the volume of the entire transducer without affecting the service life of the transducer.

With reference to the accompanying drawings, the disclosure of the present application will become easier to understand. Those skilled in the art would easily understand that these drawings are for the purpose of illustration, and are not intended to limit the protection scope of the present application. In addition, in the figures, similar numerals are used to denote similar components, where:
FIG 1 shows a perspective view of a carbon monoxide transducer;
FIG 2 shows an exploded view of a carbon monoxide transducer;
FIG 3 shows a sectional view of a carbon monoxide transducer before edge curling encapsulation; and
FIG 4 shows a sectional view of a carbon monoxide transducer after edge curling encapsulation.

It is easy to understand that, according to the technical solutions of the present invention, those skilled in the art can propose multiple replaceable structural modes and implementations. Therefore, the specific embodiments and accompanying drawings below are only exemplary descriptions of the technical solutions of the present invention, and should not be regarded as the entirety of the present invention or as limitations or restrictions on the technical solutions of the present invention.

Orientation terms such as upper, lower, left, right, front, rear, front, back, top, bottom, etc. mentioned or possibly mentioned in this specification are defined relative to the configurations illustrated in the respective drawings. They are relative concepts, so they may change accordingly according to their different locations and different states of use. Therefore, these and other orientation terms shall not be construed as restrictive terms.

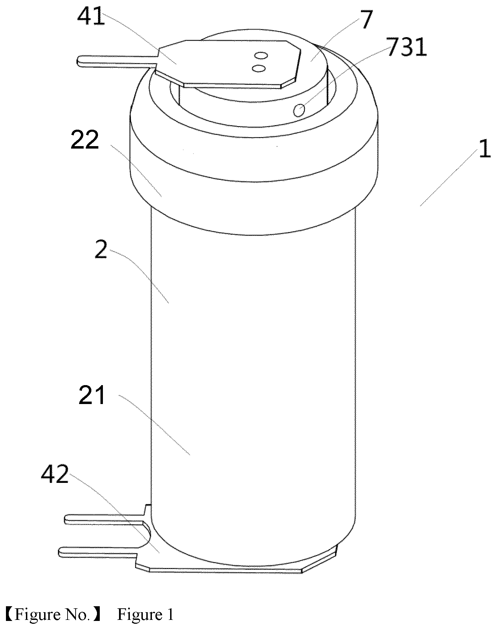

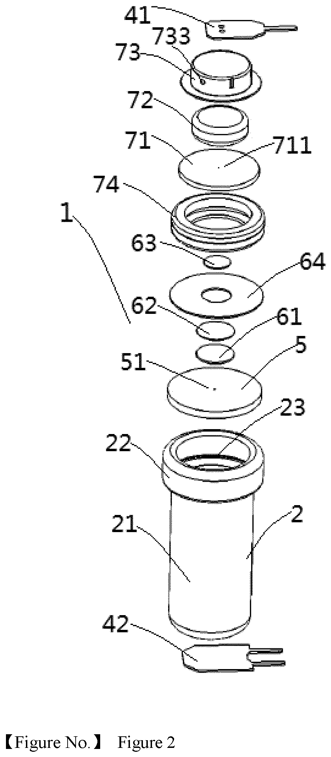

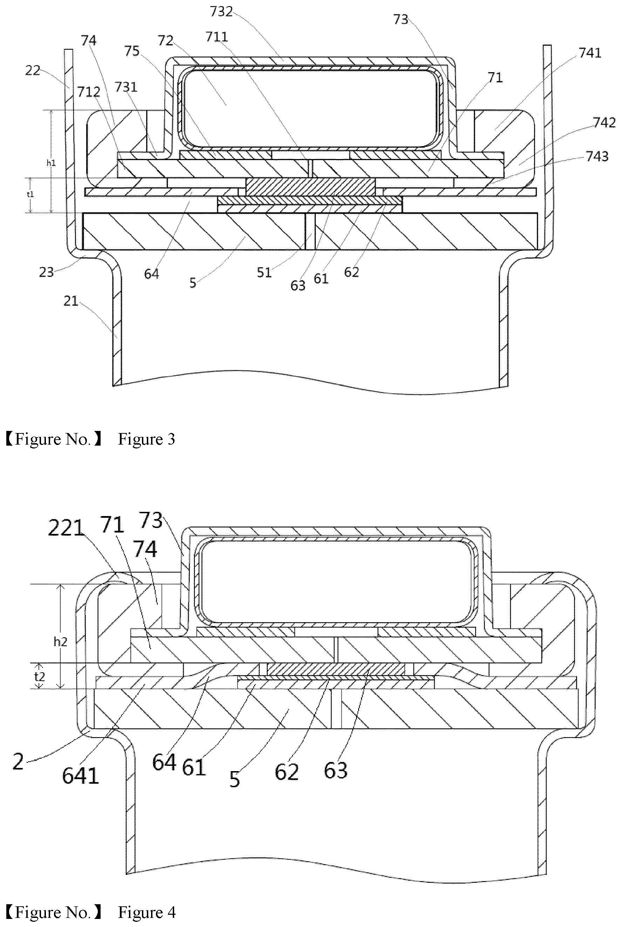

Referring to FIGS. 1 to 4, a carbon monoxide transducer is described. The carbon monoxide transducer 1 comprises: a main housing 2; a lower support plate 5 arranged in the main housing 2, wherein the lower support plate 5 divides the main housing into a water chamber containing water below the lower support plate 5 and a reaction chamber above the lower support plate 5, and the lower support plate 5 is provided with an opening 51 to allow the water in the water chamber to enter the reaction chamber; a multilayer film assembly arranged in the reaction chamber on the lower support plate 5; and a cover assembly 7 on the multilayer film assembly. The cover assembly 7 comprises: a top cover 73, an upper support plate 71, a filter body 72 between the top cover 73 and the upper support plate 71, and a sealing ring 74 wrapped around the outer periphery of the top cover 73 and the upper support plate 71. The sealing ring 74 is, for example, a rubber sealing ring, such as natural rubber. The top cover 73 is provided with an opening 733 to allow the surrounding gas to enter the cover assembly 7, and then the gas is filtered by the filter body 72 and enters the reaction chamber through an opening 711 of the upper support plate 71. As shown in FIG 4, the top of the main housing 2 is formed as a curling portion 221, which is arranged to press the sealing ring 74 of the cover assembly and the multilayer film assembly towards the lower support plate 5. Through this structure, the carbon monoxide transducer 1 according to the embodiment of the present invention has stronger sealing performance, which makes it more capable of maintaining water, so that it is more difficult for the water in the water chamber to evaporate. Accordingly, the volume of the water chamber can be smaller, so that the volume of the entire carbon monoxide transducer is significantly reduced as well. Compared with the common carbon monoxide transducers, the volume of the carbon monoxide transducer according to the present invention can be reduced by up to 70%. The reduction in volume of the carbon monoxide transducer can save material costs, such as reducing the use of materials for the respective layers of the multilayer film assembly, reduce the entry of smoke and other interfering substances, and promote the integration of the carbon monoxide transducer and the printed circuit board (PCB).

In some embodiments, the curling portion 221 is arranged to compress the sealing ring 74 and the multilayer film assembly, so that the total thickness of the sealing ring 74 and the multilayer film assembly is reduced by 20% to 40%. Specifically, as shown in FIGS. 3 and 4, the sealing ring 74 and the multilayer film assembly have a total thickness of h1 (which is defined as the distance between the upper surface of the sealing ring 74 and the lower surface of the multilayer film assembly) in an uncompressed free state, and have a total thickness of h2 in a state after being compressed by the curling portion 221, wherein h2/h1 is in the range of 0.6 to 0.8. In some embodiments, the sealing ring 74 is made of natural rubber. In some embodiments, the curling portion 221 is arranged to compress the sealing ring 74 and the multilayer film assembly, so that the total thickness of the sealing ring 74 and the multilayer film assembly is reduced by 25% to 35%, that is, h2/h1 is in the range of 0.65 to 0.75. In some embodiments, the thickness of the multilayer film assembly is reduced by at least 40%. For example, the thickness of the multilayer film assembly is reduced by at least 50%, or the thickness of the multilayer film assembly is reduced by about 60%. Specifically, as shown in FIG 3, the total thickness of the multilayer film assembly in a free state is t1 (which is defined as the distance between the upper surface and the lower surface of the multilayer film assembly). And, as shown in FIG 4, the total thickness of the multilayer film assembly in an assembled state is t2, where t2/t1 is less than 0.6. For example, t2/t1 is less than 0.5, or t2/t1 is about 0.4. The sealing ring 74 and the multilayer film assembly are made of materials with some elasticity, and the sealing performance is enhanced by pressing and deforming them. When selecting material for the sealing ring 74, one also needs to consider the capability of providing sufficient insulation and the aging resistance performance under long-term compression. Natural rubber can meet the above requirements.

The multilayer film assembly comprises: a first insulating diffusion layer 61 on the lower support plate 5, a reaction film 62 on the first insulating diffusion layer 61, a ring washer 64 on the reaction film 62, and a second insulating diffusion layer 63. The second insulating diffusion layer 63 is located in the middle of the ring washer 64, i.e., at the opening in the middle thereof. The outer diameter of the ring washer 64 is close to the inner diameter of the reaction chamber, so that the position of the second insulating diffusion layer 63 can be determined. In some embodiments, the first insulating diffusion layer 61 and the second insulating diffusion layer 63 may be made of the same material, which provides insulation and allows the passage of gas and water. The reaction film 62 may be a platinized electrode film. In some embodiments, the carbon monoxide transducer also comprises electrode sheets 41, 42 attached to the top cover 73 and the bottom of the main housing 2 respectively. When there is carbon monoxide in the environment, the carbon monoxide gas enters the multilayer film assembly from the upper side of the multilayer film assembly and water enters from the lower side of the multilayer film assembly, and reacts in the reaction film 62, resulting in a potential difference between the upper support plate 71 and the lower support plate 5. The upper support plate 71 and the top cover 73 are both made of conductive materials, such as stainless steel, and the lower support plate 5 and the main housing 2 are both made of conductive materials, such as stainless steel. Since the upper support plate 71 and the top cover 73 are connected to each other and the lower support plate 5 and the main housing 2 are connected to each other, a voltage signal is generated between the electrode sheet 41 on the top cover 73 and the electrode sheet 42 at the bottom of the main housing 2.

As shown in FIG 4, in an assembled state, the upper support plate 71 abuts against the second insulating diffusion layer 63, that is, the lower surface of the upper support plate 71 is pressed against the upper surface of the second insulating diffusion layer 63. And, the sealing ring 74 abuts against the ring washer 64, that is, the lower surface of the sealing ring 74 is pressed against the upper surface of the ring washer 64, and the outer edge of the ring washer 64 contacts the lower support plate 5. Specifically, as shown in FIG 4, the ring washer is pressed into the following state: the outer side 641 of the ring washer is between the lower support plate 5 and the sealing ring 74, the inner side of the ring washer is between the reaction film 62 and the upper support plate 71, and the middle part of the ring washer is deformed. Through this arrangement, the curling portion 221 of the main housing 2 can compress the cover assembly and the entire multilayer film assembly.

As shown in FIG 1, the main housing 2 may include an upper part 22 and a lower part 21, where the upper part 22 has a diameter slightly larger than that of the lower part 21. And, the inner side of the main housing 2 has a shoulder 23 between the upper part 22 and the lower part 21, which can be formed in the process of stamping the upper part 22 and the lower part 21 of the housing. As shown in FIG 3, the lower support plate 5 can be directly arranged on the shoulder 23 of the main housing, and is connected with the main housing at the position of the shoulder 23. In alternative embodiments, the shoulder 23 may be implemented in other forms. In alternative embodiments, the lower support plate 5 can also be fixed in the main housing 2 by other means.

In some embodiments, the top cover 73 includes a middle protruding portion 732 and a plane portion 731 at the outside of the middle protruding portion 732. The upper support plate 71 is formed as a flat plate. The plane portion 731 of the top cover abuts against the upper support plate 71, and the outer periphery is wrapped by the sealing ring 74. In some embodiments, the cross section of the sealing ring 74 is basically U-shaped, including an upper portion 741 located above the plane portion 731 of the top cover, the plane portion 731 of the top cover, a middle portion 742 at the outside of the upper support plate 71, and a lower portion 743 below the upper plane plate 71, where the upper portion 741 may have a greater thickness than the lower portion 743, and the upper portion 741 may have a larger inner diameter than the lower portion 743. There is a gap between the inner side of the upper portion 741 and the side wall of the protruding portion 732 of the top cover, so as to allow the entirety of the top cover 73, the upper support plate 71 and the filter body 72 to be assembled into the sealing ring 74 from above. During the manufacturing process, the top cover 73 and the upper support plate 71 can be welded along their edges to achieve the conductivity between the two, with the filter body 72 and a gasket 75 contained therein, and the entirety thus formed is then embedded into the sealing ring 74 (utilizing the elasticity of the sealing ring 74) from above the sealing ring 74, thus forming the top cover assembly. Then, the lower support plate, the multilayer film assembly and the top cover assembly are placed in the main housing 2, and a curling operation is performed, with the shape and pressing extent of the curling portion 221 being controlled, so as to achieve a carbon monoxide transducer with good sealing performance. Therefore, the carbon monoxide transducer according to the present invention can be easily manufactured based on the above steps.

In some embodiments, the filter body 72 is an activated carbon bag located in the chamber defined by the protruding portion 732 of the top cover. In some embodiments, a gasket 75 is also arranged between the filter body 72 and the upper support plate 71. Since an air hole 711 is processed using laser on the upper support plate 71, the gasket 75 in the middle is provided to prevent the processing burr from puncturing the filter body 72. In some embodiments, the water chamber can be defined by the interior of the lower part 21 of the main housing 2. In some embodiments, the diameter of the water chamber is less than 15mm and the height of the water chamber is less than 30mm, thus having a smaller volume than the existing structure.

The specific embodiments of the present application described above are merely intended to describe the principles of the present invention more clearly, wherein various components are clearly shown or described to facilitate the understanding of the principles of the present invention.

## Claims

1. A carbon monoxide transducer (1), comprising:
a main housing (2);
a lower support plate (5) arranged in the main housing (2), wherein the lower support plate (5) divides the main housing (2) into a water chamber containing water below the lower support plate (5) and a reaction chamber above the lower support plate;
a multilayer film assembly arranged in the reaction chamber on the lower support plate (5); and
a cover assembly (7) on the multilayer film assembly, wherein the cover assembly (7) comprises a top cover (73), an upper support plate (71), a filter body (72) between the top cover (73) and the upper support plate (71), and a sealing ring (74) wrapped around an outer periphery of the top cover (73) and the upper support plate (71);
wherein, a curling portion (221) is formed at the top of the main housing (2) that compresses the sealing ring (74) of the cover assembly (7) and the multilayer film assembly towards the lower support plate (5),
**characterised in that** the multilayer film assembly comprises a first insulating diffusion layer (61) on the lower support plate (5) and a reaction film (62) on the first insulating diffusion layer (61), a ring washer (64) on the reaction film (62), and a second insulating diffusion layer (63), wherein the second insulating diffusion layer (63) is located in a center hole of the ring washer (64), and an outer diameter of the ring washer (64) is close to an inner diameter of the reaction chamber, and
wherein the upper support plate (71) abuts against the second insulating diffusion layer (63), the bottom of the sealing ring (74) abuts against the ring washer (64), and an outer edge of the ring washer (64) is pressed down to abut against the lower support plate (5).

2. The carbon monoxide transducer (1) according to claim 1, wherein the sealing ring (74) is made of natural rubber, and the curling portion (221) is arranged to compress the sealing ring (74) and the multilayer film assembly, so that a total thickness of the sealing ring (74) and the multilayer film assembly is compressed by 20% to 40%.

3. The carbon monoxide transducer (1) according to claim 1 or 2, wherein the curling portion (221) is arranged to compress the sealing ring (74) and the multilayer film assembly, so that the total thickness of the sealing ring (74) and the multilayer film assembly is compressed by 25% to 35%.

4. The carbon monoxide transducer (1) according to any of claims 1, wherein the total thickness of the multilayer film assembly is compressed by at least 40%.

5. The carbon monoxide transducer (1) according to any preceding claim, wherein an inner side of the main housing (2) has a shoulder (23), the lower support plate (5) is arranged on the shoulder (23) of the main housing (2), and the lower support plate (5) and the main housing (2) are made of conductive materials and are connected to each other.

6. The carbon monoxide transducer (1) according to any preceding claim, wherein the top cover (73) comprises a middle protruding portion (732) and a plane portion (731) at the outside of the middle protruding portion (732), the upper support plate (71) is formed as a flat plate, the plane portion (731) of the top cover (73) abuts against the upper support plate (71), and the outer periphery is wrapped by the sealing ring (74), and wherein the top cover (73) and the upper support plate (71) are made of conductive materials and are connected to each other, the filter body (72) is located in a chamber defined by the protruding portion (732) of the top cover (73), the filter body (72) is an activated carbon bag, and a gasket (75) is further arranged between the filter body (72) and the upper support plate (71).

7. The carbon monoxide transducer (1) according to any preceding claim, further comprising: electrode sheets (41, 42) that are attached to the top cover (73) and the bottom of the main housing (2) respectively.

8. The carbon monoxide transducer (1) according to any preceding claim, wherein a diameter of the water chamber is less than 15mm and a height of the water chamber is less than 30mm.

## Patentansprüche

1. Kohlenmonoxidwandler (1), umfassend:
ein Hauptgehäuse (2);
eine im Hauptgehäuse (2) angeordnete untere Stützplatte (5), wobei die untere Stützplatte (5) das Hauptgehäuse (2) in eine Wasserkammer unter der unteren Stützplatte (5) und eine Reaktionskammer oberhalb der unteren Stützplatte;
eine in der Reaktionskammer an der unteren Stützplatte (5) angeordnete Mehrschichtfolienbaugruppe; und
eine Abdeckungsbaugruppe (7) auf der Mehrschichtfolienbaugruppe aufteilt, wobei die Abdeckungsbaugruppe (7) eine obere Abdeckung (73), eine obere Stützplatte (71), einen Filterkörper (72) zwischen der oberen Abdeckung (73) und der oberen Stützplatte (71) und einen um einen äußeren Umfang der oberen Abdeckung (73) und der oberen Stützplatte (71) gewickelten Dichtungsring (74) umfasst;
wobei an der Oberseite des Hauptgehäuses (2) ein Krümmungsabschnitt (221) ausgebildet ist, der den Dichtungsring (74) der Abdeckungsbaugruppe (7) und der Mehrschichtfolienbaugruppe in Richtung der unteren Stützplatte (5) zusammendrückt,
**dadurch gekennzeichnet, dass** die Mehrschichtfolienbaugruppe eine erste isolierende Diffusionsschicht (61) auf der unteren Stützplatte (5) und eine Reaktionsfolie (62) auf der ersten isolierenden Diffusionsschicht (61), eine Ringscheibe (64) auf der Reaktionsfolie (62) und eine zweite isolierende Diffusionsschicht (63) umfasst,
wobei sich die zweite isolierende Diffusionsschicht (63) in einem Mittelloch der Ringscheibe (64) befindet, und ein Außendurchmesser der Ringscheibe (64) nahe einem Innendurchmesser der Reaktionskammer liegt, und
wobei die obere Stützplatte (71) an der zweiten isolierenden Diffusionsschicht (63) anliegt, die Unterseite des Dichtungsrings (74) an der Ringscheibe (64) anliegt und eine Außenkante der Ringscheibe (64) nach unten gedrückt wird, um an der unteren Stützplatte (5) anzuliegen.

2. Kohlenmonoxidwandler (1) nach Anspruch 1, wobei der Dichtungsring (74) aus Naturkautschuk besteht und der Krümmungsabschnitt (221) so angeordnet ist, dass er den Dichtungsring (74) und die Mehrschichtfolienbaugruppe komprimiert, so dass eine Gesamtdicke des Dichtungsrings (74) und der Mehrschichtfolienbaugruppe um 20 % bis 40 % komprimiert wird.

3. Kohlenmonoxidwandler (1) nach Anspruch 1 oder 2, wobei der Krümmungsabschnitt (221) so angeordnet ist, dass er den Dichtungsring (74) und die Mehrschichtfolienbaugruppe komprimiert, so dass die Gesamtdicke des Dichtungsrings (74) und der Mehrschichtfolienbaugruppe um 25 % bis 35 % komprimiert wird.

4. Kohlenmonoxidwandler (1) nach einem der Ansprüche 1, wobei die Gesamtdicke der Mehrschichtfolienbaugruppe um mindestens 40 % komprimiert wird.

5. Kohlenmonoxidwandler (1) nach einem der vorhergehenden Ansprüche, wobei eine Innenseite des Hauptgehäuses (2) eine Schulter (23) aufweist, die untere Stützplatte (5) auf der Schulter (23) des Hauptgehäuses (2) angeordnet ist und die untere Stützplatte (5) und das Hauptgehäuse (2) aus leitfähigen Materialien bestehen und miteinander verbunden sind.

6. Kohlenmonoxidwandler (1) nach einem der vorhergehenden Ansprüche, wobei die obere Abdeckung (73) einen mittleren vorstehenden Abschnitt (732) und einen ebenen Abschnitt (731) an der Außenseite des mittleren vorstehenden Abschnitts (732) umfasst, wobei die obere Stützplatte (71) als flache Platte ausgebildet ist, der ebene Teil (731) der oberen Abdeckung (73) an der oberen Stützplatte (71) anliegt, und der äußere Umfang von dem Dichtungsring (74) umwickelt ist, und wobei die obere Abdeckung (73) und die obere Stützplatte (71) aus leitfähigen Materialien bestehen und miteinander verbunden sind, der Filterkörper (72) sich in einer Kammer befindet, die durch den vorstehenden Teil (732) der oberen Abdeckung (73) definiert ist, der Filterkörper (72) ein Aktivkohlebeutel ist und eine Dichtung (75) weiter zwischen dem Filterkörper (72) und der oberen Stützplatte (71) angeordnet ist.

7. Kohlenmonoxidwandler (1) nach einem der vorhergehenden Ansprüche, weiter umfassend: Elektrodenbleche (41, 42), die jeweils an der oberen Abdeckung (73) und der Unterseite des Hauptgehäuses (2) befestigt sind.

8. Kohlenmonoxidwandler (1) nach einem der vorhergehenden Ansprüche, wobei ein Durchmesser der Wasserkammer weniger als 15 mm und eine Höhe der Wasserkammer weniger als 30 mm beträgt.

## Revendications

1. Transducteur (1) de monoxyde de carbone, comprenant :
un logement principal (2) ;
une plaque de support inférieure (5) agencée dans le logement principal (2), dans lequel la plaque de support inférieure (5) divise le logement principal (2) en une chambre d'eau contenant de l'eau sous la plaque de support inférieure (5) et une chambre de réaction au-dessus de la plaque de support inférieure ;
un ensemble film multicouche agencé dans la chambre de réaction sur la plaque de support inférieure (5) ; et
un ensemble couvercle (7) sur l'ensemble film multicouche, dans lequel l'ensemble couvercle (7) comprend un couvercle haut (73), une plaque de support supérieure (71), un corps (72) de filtre entre le couvercle haut (73) et la plaque de support supérieure (71) et une bague (74) d'étanchéité enveloppée autour d'une périphérie extérieure du couvercle haut (73) et de la plaque de support supérieure (71) ;
dans lequel une portion de recourbement (221) est formée en haut du logement principal (2) qui comprime la bague (74) d'étanchéité de l'ensemble couvercle (7) et de l'ensemble film multicouche vers la plaque de support inférieure (5),
**caractérisé en ce que** l'ensemble film multicouche comprend une première couche de diffusion isolante (61) sur la plaque de support inférieure (5) et un film (62) de réaction sur la première couche de diffusion isolante (61), une rondelle (64) sur le film (62) de réaction, et une deuxième couche de diffusion isolante (63),
dans lequel la deuxième couche de diffusion isolante (63) est située dans un trou central de la rondelle (64), et un diamètre extérieur de la rondelle (64) est proche d'un diamètre intérieur de la chambre de réaction, et
dans lequel la plaque de support supérieure (71) vient en butée contre la deuxième couche de diffusion isolante (63), le fond de la bague (74) d'étanchéité vient en butée contre la rondelle (64), et un bord extérieur de la rondelle (64) est pressé vers le bas pour venir en butée contre la plaque de support inférieure (5).

2. Transducteur (1) de monoxyde de carbone selon la revendication 1, dans lequel la bague (74) d'étanchéité est en caoutchouc naturel, et la portion de recourbement (221) est agencée pour comprimer la bague (74) d'étanchéité et l'ensemble film multicouche, de sorte qu'une épaisseur totale de la bague (74) d'étanchéité et de l'ensemble film multicouche soit comprimée de 20 % à 40 %.

3. Transducteur (1) de monoxyde de carbone selon la revendication 1 ou la revendication 2, dans lequel la portion de recourbement (221) est agencée pour comprimer la bague (74) d'étanchéité et l'ensemble film multicouche, de sorte que l'épaisseur totale de la bague (74) d'étanchéité et de l'ensemble film multicouche soit comprimée de 25 % à 35 %.

4. Transducteur (1) de monoxyde de carbone selon l'une quelconque des revendications 1, dans lequel l'épaisseur totale de l'ensemble film multicouche est comprimée d'au moins 40 %.

5. Transducteur (1) de monoxyde de carbone selon une quelconque revendication précédente, dans lequel un côté intérieur du logement principal (2) présente un épaulement (23), la plaque de support inférieure (5) est agencée sur l'épaulement (23) du logement principal (2), et la plaque de support inférieure (5) et le logement principal (2) sont en matériaux conducteurs et sont reliés l'un à l'autre.

6. Transducteur (1) de monoxyde de carbone selon une quelconque revendication précédente, dans lequel le couvercle haut (73) comprend une portion saillante centrale (732) et une portion plane (731) à l'extérieur de la portion saillante centrale (732), la plaque de support supérieure (71) est formée sous forme de plaque plate, la portion plane (731) du couvercle haut (73) vient en butée contre la plaque de support supérieure (71), et la périphérie extérieure est enveloppée par la bague (74) d'étanchéité, et dans lequel le couvercle haut (73) et la plaque de support supérieure (71) sont en matériaux conducteurs et sont reliés l'un à l'autre, le corps (72) de filtre est situé dans une chambre définie par la portion saillante (732) du couvercle haut (73), le corps (72) de filtre est un sac de charbon actif, et un joint (75) est en outre agencé entre le corps (72) de filtre et la plaque de support supérieure (71).

7. Transducteur (1) de monoxyde de carbone selon une quelconque revendication précédente, comprenant en outre : des feuilles (41, 42) d'électrode qui sont fixées au couvercle haut (73) et au fond du logement principal (2) respectivement.

8. Transducteur (1) de monoxyde de carbone selon une quelconque revendication précédente, dans lequel un diamètre de la chambre d'eau est de moins de 15 mm et une hauteur de la chambre d'eau est de moins de 30 mm.
